(19) Europäisches Patentamt / European Patent Office / Office européen des brevets

(11) **EP 3 520 675 A1**

(12) **EUROPEAN PATENT APPLICATION**
published in accordance with Art. 153(4) EPC

(43) Date of publication:
**07.08.2019 Bulletin 2019/32**

(51) Int Cl.:
***A61B 1/045*** (2006.01)  ***A61B 1/00*** (2006.01)
***G02B 23/24*** (2006.01)  ***H04N 7/18*** (2006.01)

(21) Application number: **17855372.3**

(22) Date of filing: **18.07.2017**

(86) International application number:
**PCT/JP2017/025877**

(87) International publication number:
**WO 2018/061414 (05.04.2018 Gazette 2018/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **29.09.2016 JP 2016192057**

(71) Applicant: **Fujifilm Corporation**
**Minato-ku**
**Tokyo 106-8620 (JP)**

(72) Inventor: **SAITO Takaaki**
**Ashigarakami-gun**
**Kanagawa 258-8538 (JP)**

(74) Representative: **Klunker IP**
**Patentanwälte PartG mbB**
**Destouchesstraße 68**
**80796 München (DE)**

(54) **ENDOSCOPE SYSTEM, PROCESSOR DEVICE, AND ENDOSCOPE SYSTEM OPERATING METHOD**

(57)    Provided are an endoscopic system, a processor device, and a method of operating an endoscopic system that accurately index the depth of blood vessels within tissue of an observation object irrespective of the scattering characteristics of the observation object. A blood vessel depth feature amount calculation unit (72) calculates a blood vessel depth feature amount (Z). A scattering characteristics feature amount calculation unit (74) calculates a scattering characteristics feature amount (W) in which the blood vessel depth feature amount is not included and scattering characteristics in an observation object are reflected. A feature amount correction unit (76) corrects the blood vessel depth feature amount (Z) depending on the scattering characteristics feature amount (W) to obtain a corrected blood vessel depth feature amount (Za). A display unit (18) displays a blood vessel depth image on the basis of the corrected blood vessel depth feature amount (Za).

FIG. 7

EP 3 520 675 A1

**Description**

**BACKGROUND OF THE INVENTION**

1. Field of the Invention

The present invention relates to an endoscopic system, a processor device, and a method of operating an endoscopic system that indexes the depth of blood vessels within tissue of an observation object.

2. Description of the Related Art

In the medical field, diagnosis using an endoscopic system including a light source device, an endoscope, and a processor device has been performed widely. In the medical diagnosis using the endoscopic system, an insertion part of the endoscope is inserted into a subject and an observation object is irradiated with illumination light from a distal end part of the endoscope. Then, the observation object under the irradiation with the illumination light is imaged by an imaging sensor of the distal end part, and an image of the observation object is generated using obtained image signals and displayed on a monitor.

Additionally, in recent years, diagnosing a lesion location is performed paying attention to blood vessel patterns having different depths in tissue, such as surface layer blood vessels or extreme surface layer blood vessels. In JP2016-067775A, an observation object is illuminated with two-wavelengths illumination light having mutually different scattering coefficients, and blood vessels having different depths are displayed in different color tones, respectively on the basis of a two-wavelengths image obtained from the two-wavelengths illumination light.

**SUMMARY OF THE INVENTION**

In JP2016-067775A, a blood vessel depth feature amount (equivalent to a "computed image signal" in JP2016-067775A) obtained by indexing the depth of the blood vessels within tissue is calculated by using a difference in scattering coefficient in the two-wavelengths illumination light. However, in a case where scattering characteristics of light in the observation object vary due to various factors, the blood vessel depth feature amount also varies in accordance with the scattering characteristics that have varied. In this way, in a case where the blood vessel depth feature amount varies, even blood vessels at the same depth may be displayed in different color tones.

An object of the invention is to provide an endoscopic system, a processor device, and a method of operating an endoscopic system that accurately index the depth of blood vessels within tissue of an observation object irrespective of the scattering characteristics of the observation object.

In order to achieve the above object, an endoscopic system of the invention comprises a blood vessel depth feature amount calculation unit that calculates a blood vessel depth feature amount; a scattering characteristics feature amount calculation unit that calculates a scattering characteristics feature amount in which the blood vessel depth feature amount is not included and scattering characteristics in an observation object are reflected; a feature amount correction unit that corrects the blood vessel depth feature amount depending on the scattering characteristics feature amount to obtain a corrected blood vessel depth feature amount; and a display unit that displays a blood vessel depth image on the basis of the corrected blood vessel depth feature amount.

It is preferable that the endoscopic system further comprises an image acquisition unit that acquires a first image, a second image, and a third image that have mutually different wavelength information, the blood vessel depth feature amount calculation unit calculates the blood vessel depth feature amount by performing computation based on the first image and the second image, and the scattering characteristics feature amount calculation unit calculates the scattering characteristics feature amount by performing computation based on the second image and the third image. It is preferable that a scattering coefficient of the observation object in a wavelength range of the first image is different from a scattering coefficient of the observation object in a wavelength range of the second image. It is preferable that the blood vessel depth feature amount is a ratio between the first image and the second image or a difference between the first image and the second image.

It is preferable that the scattering characteristics feature amount calculation unit generates a specific image including wavelength information in a first specific wavelength range of 430 to 520 nm, on the basis of the second image and the third image, and calculates the scattering characteristics feature amount on the basis of the specific image. It is preferable that the second image has wavelength information of a shorter wavelength than the first specific wavelength range, and the third image has wavelength information of a longer wavelength than the first specific wavelength range. It is preferable that the endoscopic system further comprises a light source that emits at least blue light and green light toward the observation object, and wavelength information corresponding to the blue light is included in the second image, and wavelength information corresponding to the green light is included in the third image.

It is preferable that the scattering characteristics feature amount calculation unit generates a specific image including wavelength information in a second specific wavelength range of 480 to 520 nm, on the basis of the second image and the third image, and calculates the scattering characteristics feature amount on the basis of the specific image. It is preferable that the endoscopic system further comprises a light source that emits at least green light toward the observation object, and wavelength information corresponding to light in the second specific wavelength range in the green light is included in the second image, and wavelength information corresponding to the green light is included in the third image. It is preferable that an oxygen saturation feature amount is not included in the scattering characteristics feature amount.

It is preferable that the feature amount correction unit obtains the corrected blood vessel depth feature amount by performing a correction in which a correction value obtained by multiplying the scattering characteristics feature amount by a certain coefficient is subtracted from the blood vessel depth feature amount. It is preferable that the endoscopic system further comprises a resolution reduction processing unit that reduces resolution of the corrected blood vessel depth feature amount; and an image generation unit that allocates either the first image or the second image to a brightness channel Y and allocates the resolution-reduced corrected blood vessel depth image to color difference channels Cr and Cb to generate the blood vessel depth image.

An endoscopic system of the invention comprises an image acquisition unit that acquires a first image, a second image, and a third image that have mutually different wavelength information; a blood vessel depth feature amount calculation unit that calculates a blood vessel depth feature amount by performing computation based on the first image and the second image; a scattering characteristics feature amount calculation unit that calculates a scattering characteristics feature amount in which the blood vessel depth feature amount is not included and scattering characteristics in an observation object are reflected, the scattering characteristics feature amount calculation unit calculating the scattering characteristics feature amount by performing computation based on the second image and the third image; a feature amount correction unit that obtains a corrected blood vessel depth feature amount by performing a correction in which a correction value obtained by multiplying the scattering characteristics feature amount by a certain coefficient is subtracted from the blood vessel depth feature amount; a resolution reduction processing unit that reduces resolution of the corrected blood vessel depth feature amount; an image generation unit that allocates either the first image or the second image to a brightness channel Y and allocates the resolution-reduced corrected blood vessel depth image to color difference channels Cr and Cb to generate a blood vessel depth image; and a display unit that displays the blood vessel depth image.

A processor device of the invention comprises a blood vessel depth feature amount calculation unit that calculates a blood vessel depth feature amount; a scattering characteristics feature amount calculation unit that calculates a scattering characteristics feature amount in which the blood vessel depth feature amount is not included and scattering characteristics in an observation object are reflected; and a feature amount correction unit that corrects the blood vessel depth feature amount depending on the scattering characteristics feature amount to obtain a corrected blood vessel depth feature amount.

A method of operating an endoscopic system of the invention comprises a step of calculating a blood vessel depth feature amount, using a blood vessel depth feature amount calculation unit; a step of calculating a scattering characteristics feature amount in which the blood vessel depth feature amount is not included and scattering characteristics in an observation object are reflected, using a scattering characteristics feature amount calculation unit; a step of correcting the blood vessel depth feature amount depending on the scattering characteristics feature amount to obtain a corrected blood vessel depth feature amount, using a feature amount correction unit; and a step of displaying a blood vessel depth image on the basis of the corrected blood vessel depth feature amount, using a display unit.

According to the invention, the depth of the blood vessels within the tissue of the observation object can be accurately indexed irrespective of the scattering characteristics of the observation object.

## BRIEF DESCRIPTION OF THE DRAWINGS

Fig. 1 is an external view of an endoscopic system.
Fig. 2 is a block diagram illustrating the functions of the endoscopic system.
Fig. 3 is a graph illustrating the spectroscopic spectrum of purple light V, blue light B, green light G, and red light R.
Fig. 4 is an explanatory view illustrating a first light emission pattern and a second light emission pattern that are performed in a first embodiment.
Fig. 5 is a graph that illustrates a relationship between a scattering coefficient and wavelength.
Fig. 6 is a graph illustrating spectral characteristics of color filters.
Fig. 7 is a block diagram illustrating functions of a special image processing unit.
Fig. 8 is a graph illustrating a relationship between blood vessel depth feature amount Z and blood vessel depth.

Fig. 9 is a graph schematically expressing a relationship between the depth of blood vessels and the contrast of the blood vessels.

Fig. 10 is an explanatory view illustrating a blood vessel depth image generating method.

Fig. 11 is a flowchart in a special observation mode.

Fig. 12 is a schematic view of a blood vessel depth image.

Fig. 13 is an explanatory view illustrating a blood vessel depth image generating method different from that Fig. 10.

Fig. 14 is a block diagram illustrating a cooperation relationship between a special image processing unit and an alignment processing unit.

Fig. 15 is an explanatory view illustrating a first light emission pattern and a second light emission pattern that are performed in a second embodiment.

Fig. 16 is an explanatory view illustrating a first light emission pattern and a second light emission pattern that are performed in a third embodiment.

Fig. 17 is a block diagram illustrating a light source of a fourth embodiment.

Fig. 18 is a schematic view illustrating a rotation filter.

Fig. 19 is a schematic view illustrating a capsule endoscope of a fifth embodiment.


## DESCRIPTION OF THE PREFERRED EMBODIMENTS

[First Embodiment]

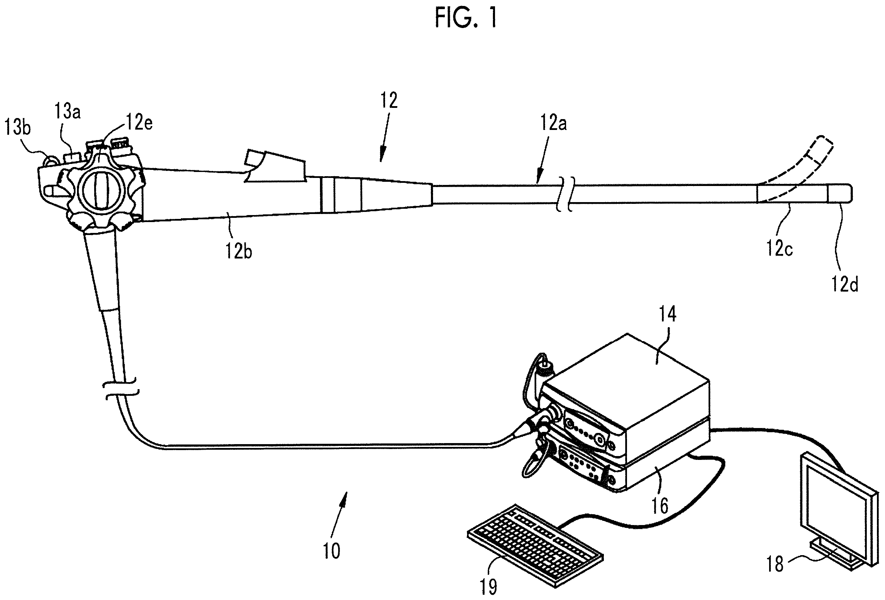

As illustrated in Fig. 1, an endoscopic system 10 has an endoscope 12, a light source device 14, a processor device 16, a monitor 18, and a console 19. The endoscope 12 is optically connected to the light source device 14 and is electrically connected to the processor device 16. The endoscope 12 has an insertion part 12a to be inserted into a subject, an operating part 12b provided at a proximal end portion of the insertion part 12a, and a bending part 12c and a distal end part 12d provided on a distal end side of the insertion part 12a. By operating an angle knob 12e of the operating part 12b, the bending part 12c makes a bending motion. The distal end part is directed in a desired direction by this bending motion.

Additionally, the operating part 12b is provided with a mode changeover switch 13a, a zooming operating part 13b, a still image acquisition instruction part (not illustrated), and the like other than the angle knob 12e. The mode changeover switch 13a is used for switching the operation of observation modes. The endoscopic system 10 has a normal observation mode and a special observation mode as the observation modes. In the normal observation mode, a natural-tone image (hereinafter, referred to as a normal image) obtained by imaging the observation object using white light for illumination light is displayed on the monitor 18. In the special observation mode, an image (hereinafter referred to as a blood vessel depth image) for displaying blood vessels at different depths in different color tones are generated and displayed using image signals obtained by imaging the observation object.

The processor device 16 is electrically connected to the monitor 18 and the console 19. The monitor 18 outputs and displays the image of the observation object, information accompanying the image of the observation object, and the like. The console 19 functions as a user interface that receives an input operation, such as a function setting. In addition, an external recording unit (not illustrated) that records the images, the image information, and the like may be connected to the processor device 16.

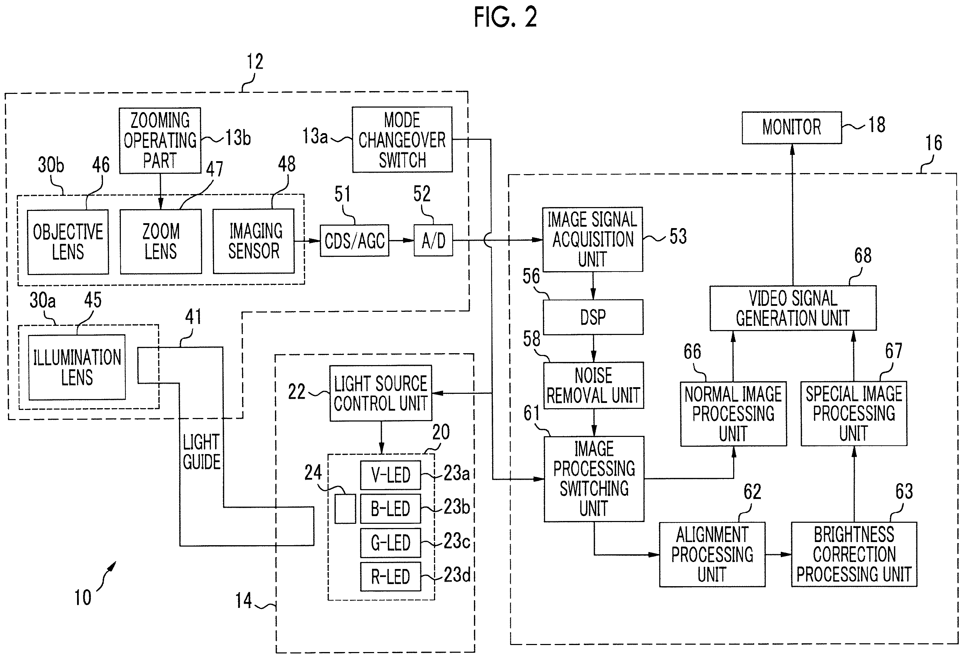

As illustrated in Fig. 2, the light source device 14 includes a light source 20, and a light source control unit 22 that controls the light source 20. The light source 20 has a plurality of semiconductor light sources, turns on or off these semiconductor light sources, respectively, and generates illumination light for irradiating the observation object by controlling the light emission amounts of the respective semiconductor light sources in a case where the semiconductor light sources are turned on. In the first embodiment, the light source 20 has a wavelength cutoff filter 24 provided at a light emitting part of the B-LED 23b in addition to four color LEDs of a purple light emitting diode (V-LED) 23a, a blue light emitting diode (B-LED) 23b, a green light emitting diode (G-LED) 23c, and a red light emitting diode (R-LED) 23d. In addition, laser diodes (LDs) may be used instead of the LEDs.

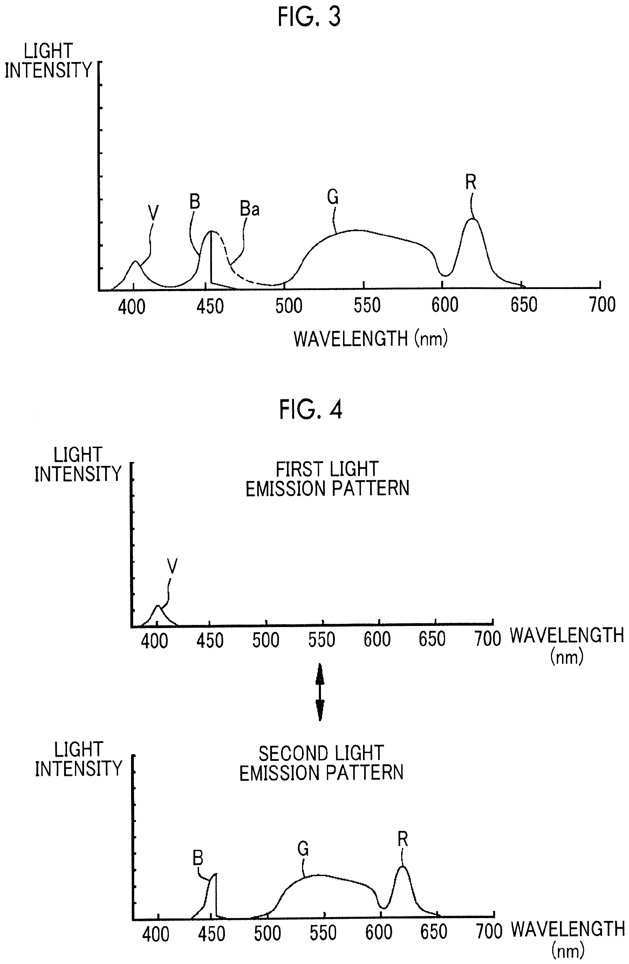

As illustrated in Fig. 3, the V-LED 23a is a purple semiconductor light source that emits purple light V having a central wavelength of 405 nm and a wavelength range of 380 to 420 nm. The B-LED 23b is a blue semiconductor light source that emits blue light Ba having a central wavelength of 445 nm and a wavelength range of 420 to 500 nm. The blue light Ba emitted from the B-LED 23b is cut by the wavelength cutoff filter 24 on at least a longer wavelength side than a peak wavelength of 450 nm. Accordingly, the blue light B after being transmitted through the wavelength cutoff filter 24 has a wavelength range of 420 to 460 nm. In this way, the reason why light in a wavelength range on the longer wavelength side than 460 nm is cut is that the light in the wavelength range on the longer wavelength side than 460 nm is a factor in which the blood vessel contrast is lowered. In addition, the wavelength cutoff filter 24 may reduce the light in the wavelength range on the longer wavelength side than 460 nm instead of cutting the light in the wavelength range on the longer wavelength side than 460 nm.

The G-LED 23c is a green semiconductor light source that emits green light G having a wavelength range of 480 to 600 nm. The R-LED 23d is a red semiconductor light source that emits red light R having a central wavelength of 620 to 630 nm and a wavelength range of 600 to 650 nm. In addition, the central wavelengths of the V-LED 23a and the B-LED 23b have a width of about ±5 nm to ±10 nm. Additionally, the central wavelength and the peak wavelength in each of the LEDs 23a to 23d may be the same as each other or different from each other.

The light source control unit 22 can individually control ON/OFF states of the LEDs 23a to 23d, the light emission amounts thereof at the time of the ON state, and the like by inputting independent control signals to the LEDs, respectively. In the case of the normal observation mode, the light source control unit 22 turns on the V-LED 23a, the B-LED 23b, the G-LED 23c, and the R-LED 23d altogether. For this reason, in a normal observation mode, white light including the purple light V, the blue light B, the green light G, and the red light R is used as the illumination light.

On the other hand, in the case of the special observation mode, the light source control unit 22 controls the light source 20 in a first light emission pattern in which only the V-LED 23a is turned on and the other LEDs, such as the B-LED 23b, are turned off, and a second light emission pattern in which the V-LED 23a is turned off, the B-LED 23b, the G-LED 23c, and the R-LED 23d are turned on. That is, in the special observation mode, as illustrated in Fig. 4, the observation object is irradiated with the purple light V in the first light emission pattern, and the observation object is irradiated with mixed color light of the blue light B, the green light G, and the red light R in the subsequent second light emission pattern. In addition, the blue light B radiated in the second light emission pattern is cut by the wavelength cutoff filter 24 on the longer wavelength side.

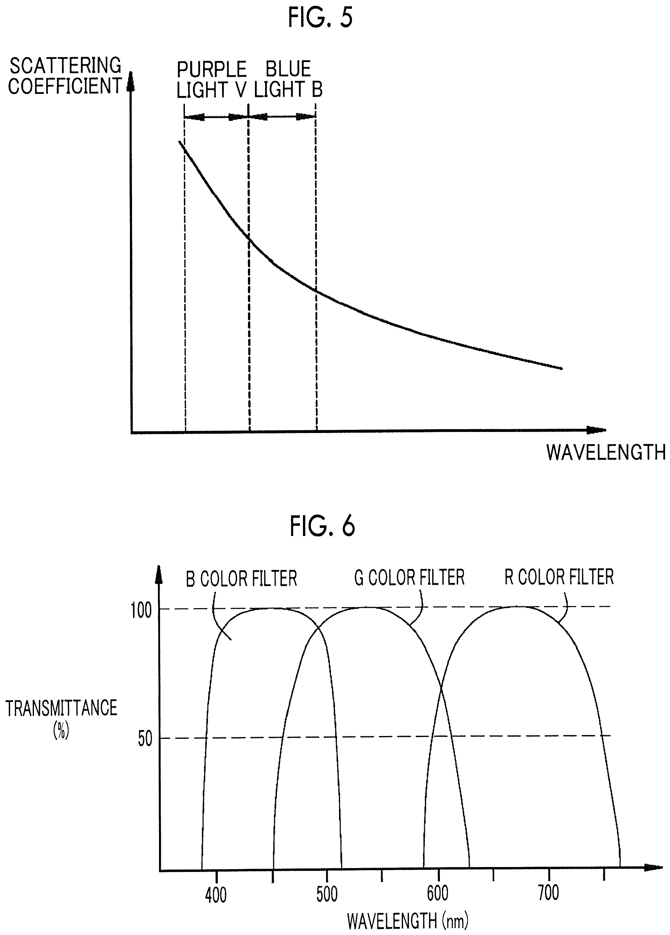

In addition, as illustrated in Fig. 5, the scattering coefficient of the observation object included in a wavelength range of the purple light V and the scattering coefficient of the observation object included in a wavelength range of the blue light B are different from each other, and are larger than the scattering coefficient of the observation object included in other wavelength ranges. The scattering coefficients of the observation object relate to the depths of reach to the observation object, that is, depths under mucous membranes of blood vessels observable in the wavelength ranges. Hence, the depth of blood vessels in the observation object can be acquired by using two kinds of illumination light including the purple light V and the blue light B. Here, the expression "the scattering coefficients are different from each other" means that the ratio of the scattering coefficients of the second illumination light to the scattering coefficient of the first illumination light is 0.8 or less. Additionally, a difference in scattering coefficient in a case where the scattering coefficients are different from each other may be 70 (cm$^{-1}$) or more.

As illustrated in Fig. 2, light of respective colors emitted from the respective LEDs 23a to 23d enters a light guide 41 inserted into the insertion part 12a via a light path coupling part (not illustrated) formed with a mirror, a lens, or the like. The light guide 41 is built in the endoscope 12 and a universal cord (a cord that connects the endoscope 12, and the light source device 14 and the processor device 16 together). The light guide 41 propagates the illumination light generated by the light source 20 up to the distal end part 12d of the endoscope 12.

The distal end part 12d of the endoscope 12 is provided with an illumination optical system 30a and an imaging optical system 30b. The illumination optical system 30a has an illumination lens 45, and the illumination light propagated by the light guide 41 is radiated to the observation object via the illumination lens 45. The imaging optical system 30b has an objective lens 46, a zoom lens 47, and an imaging sensor 48. Various kinds of light, such as reflected light, scattered light, and fluorescence from the observation object resulting from radiating illumination light, enters the imaging sensor 48 via the objective lens 46 and the zoom lens 47. Accordingly, the image of the observation object is formed on the imaging sensor 48. The zoom lens 47 is freely moved between a telephoto end and a wide end by operating the zooming operating part 13b, and magnifies or reduces a reflected image of the observation object of which the image is to be formed on the imaging sensor 48.

The imaging sensor 48 is a primary color (RGB) color imaging sensor that images the observation object irradiated with the illumination light. In each pixel of the imaging sensor 48, any one of a blue (B) color filter, a, green (G) color filter, and a red (R) color filter illustrated in Fig. 6 is provided for each pixel. The B color filter has a spectral transmittance of 380 to 520 nm. The G color filter has a spectral transmittance of 450 to 630 nm. The R color filter has a spectral transmittance of 580 to 760 nm.

The imaging sensor 48 receives blue light in a blue pixel (B pixel) provided with the blue (B) color filter, receives green light in a green pixel (G pixel) provided with the green (G) color filter, and receives red light in a red pixel (R pixel) provided with the red (R) color filter. Image signals of respective RGB colors are output from the respective color pixels. In the normal observation mode, in a case where the observation object is illuminated with white light, the imaging sensor 48 images the observation object illuminated with the white light, thereby outputting a Bc image signal from the B pixel, outputting a Gc image signal from the G pixel, and outputting an Rc image signal from the R pixel.

In the special observation mode, in a case where the observation object is illuminated with the purple light V in the first light emission pattern, the imaging sensor 48 images the observation object illuminated with the purple light V, thereby outputting a B1 image signal from the B pixel, outputting a G1 image signal from the G pixel, and outputting an R1 image signal from the R pixel. Since the purple light V radiated in the first light emission pattern is mainly received in the B pixel, wavelength information corresponding to the purple light V is included in the B1 image signal (corresponding to a "first image" of the invention). In addition, the wavelength information means subject information obtained by illuminating and imaging the observation object with light in a certain wavelength range, and means, for example, various structures, such as blood vessel and ducts, which that is observable with light in a certain wavelength range and is difficult to observe with light in other wavelength ranges.

In a case where the observation object is illuminated with the blue light B, the green light G, and the red light R in the second light emission pattern, the imaging sensor 48 images the observation object illuminated with the blue light B, the green light G, and the red light R, thereby outputting a B2 image signal from the B pixel, outputting a G2 image signal from the G pixel, and outputting an R2 image signal from the R pixel. Wavelength information corresponding to the blue light B and wavelength information corresponding to light of 480 to 560 nm in the green light G are mainly included in the B2 image signal (corresponding to a "second image" of the invention). Wavelength information corresponding to light of 450 to 500 nm in the blue light B and wavelength information corresponding to the green light G are mainly included in the G2 image signal (corresponding to a "third image" of the invention). Wavelength information corresponding to 580 to 600 nm light in the green light G and wavelength information corresponding to the red light R are mainly included in the R2 image signal.

As the imaging sensor 48, a charge coupled device (CCD) imaging sensor or a complementary metal-oxide semiconductor (CMOS) imaging sensor is available. Additionally, instead of the primary color imaging sensor 48, a complementary color imaging sensor including complementary color filters in C (cyan), M (magenta), Y (yellow), and G (green) may be used. in a case where the complementary color imaging sensor is used, image signals in four colors of CMYG are output. Thus, image signals of RGB that are the same colors as those of the imaging sensor 48 can be obtained by converting the image signals in four colors of CMYG into the image signals in three colors of RGB through color conversion of complementary color to primary color.

As illustrated in Fig. 2, the CDS/AGC circuit 51 performs correlated double sampling (CDS) and automatic gain control (AGC) on analog image signals obtained from the imaging sensor 48. The image signals that have passed through the CDS/AGC circuit 51 are converted into digital image signals by an analog-to-digital (A/D) converter 52. The digital image signals after the A/D conversion are input to the processor device 16.

The processor device 16 includes an image signal acquisition unit 53, a digital signal processor (DSP) 56, a noise removal unit 58, an image processing switching unit 61, a normal image processing unit 66, a special image processing unit 67, and a video signal generation unit 68. The image signal acquisition unit 53 (corresponding to an "image acquisition unit" of the invention) acquires digital image signals from the imaging sensor 48 via the CDS/AGC circuit 51 and an A/D converter 52. For example, the processor device 16 has a central processing unit (CPU), and the CPU functions as the image signal acquisition unit 53, the noise removal unit 58, the image processing switching unit 61, an alignment processing unit 62, a brightness correction processing unit 63, the normal image processing unit 66, the special image processing unit 67, and the video signal generation unit 68.

The DSP 56 performs various kinds of signal processing, such as defect correction processing, offset processing, gain correction processing, linear matrix processing, gamma conversion processing, demosaicing processing, and the like, on the acquired image signals. In the defect correction processing, a signal of a defective pixel of the imaging sensor 48 is corrected. In the offset processing, a dark current component is removed from image signals subjected to the defect correction processing, and an accurate zero level is set. In the gain correction processing, a signal level is adjusted by multiplying the image signals after the offset processing by a specific gain.

The linear matrix processing for enhancing color reproducibility is performed on the image signals after the gain correction processing. Then, brightness and color saturation are adjusted by the gamma conversion processing. The demosaicing processing (also referred to as equalization processing of a grade or synchronization processing) is performed on the image signals after the gamma conversion processing, and a signal of a color that runs short in each pixel is generated by interpolation. By means of this demosaicing processing, all pixels have signals of respective RGB colors. The noise removal unit 58 performs noise removal processing using (for example, a moving average method, a median filter method, or the like) on the image signals subjected to the demosaicing processing or the like by the DSP 56, and removes noise. The image signals from which noise are removed is transmitted to the image processing switching unit 61.

The image processing switching unit 61 transmits the received image signals to the normal image processing unit 66 in a case where the normal observation mode is set, and transmits the received image signals to the special image processing unit 67 in a case where the special observation mode is set.

The normal image processing unit 66 operates in a case where the normal observation mode is set, and performs color conversion processing, color enhancement processing, and structure enhancement processing on the received image signals to generate normal image signals. In the color conversion processing, color conversion processing is performed on the RGB image signals by 3x3 matrix processing, gradation transformation processing, three-dimensional look-up table (LUT) processing, and the like. The color enhancement processing is performed on the image signals subjected to the color conversion processing. The structure enhancement processing is, for example, the processing of enhancing the structure of the observation object, such as surface layer blood vessels and pit patterns, and is performed on the image signals after the color enhancement processing. As described above, a color image obtained using the normal image signals subjected to the various kinds of image processing and the like up to the structure enhancement processing is a normal image.

The special image processing unit 67 operates in a case where the special observation mode is set. In the special image processing unit 67, blood vessel depth feature amount regarding the depth of blood vessels are calculated, and a blood vessel depth feature influenced by scattering characteristics feature amount W reflecting scattering characteristics in the observation object is corrected. The alignment processing unit 62 and the brightness correction processing unit 63 are provided between the special image processing unit 67 and the image processing switching unit 61, and alignment processing and brightness correction processing of the B1 image signal and the B2 image signal used for the calculation of the blood vessel depth feature amount are performed in the alignment processing unit 62 and the brightness correction processing unit 63.

The alignment processing unit 62 performs alignment processing between an observation object indicated by the B1 image signal and an observation object indicated by the B2 image signal, which are sequentially acquired. The alignment processing unit 62 corrects at least one of the B1 image signal or the B2 image signal.

The brightness correction processing unit 63 corrects the brightness of at least one of the B1 image signal or the B2 image signal such that the brightnesses of the B1 image signal and the B2 image signal aligned by the alignment processing unit 62 have a specific ratio. Specifically, since the light quantity ratio of the purple light V in the first light emission pattern and the blue light B in the second light emission pattern is known, gain correction for correcting brightness is performed such that the brightness of the B 1 image signal is made to coincide with the brightness of the B2 image signal in order to obtain brightnesses in a case where the observation object is irradiated with the purple light V and the blue light B of respectively equal light quantities, using the light quantity ratio.

For example, the brightness correction processing unit 63 calculates the brightness of a mucous membrane of the observation object indicated by the B1 image signal by calculating an average value of pixel values of all pixels having the B1 image signal, and calculates the brightness of a mucous membranes of the observation object indicated by the B2 image signal by calculating an average value of pixel values of all pixels having the B2 image signal. Then, a gain for causing the brightness of the mucous membrane of the observation object indicated by the B1 image signal and the brightness of the mucous membrane of the observation object indicated by the B2 image signal to coincide with each other is calculated, and the brightness of the B1 image signal is corrected using the calculated gain.

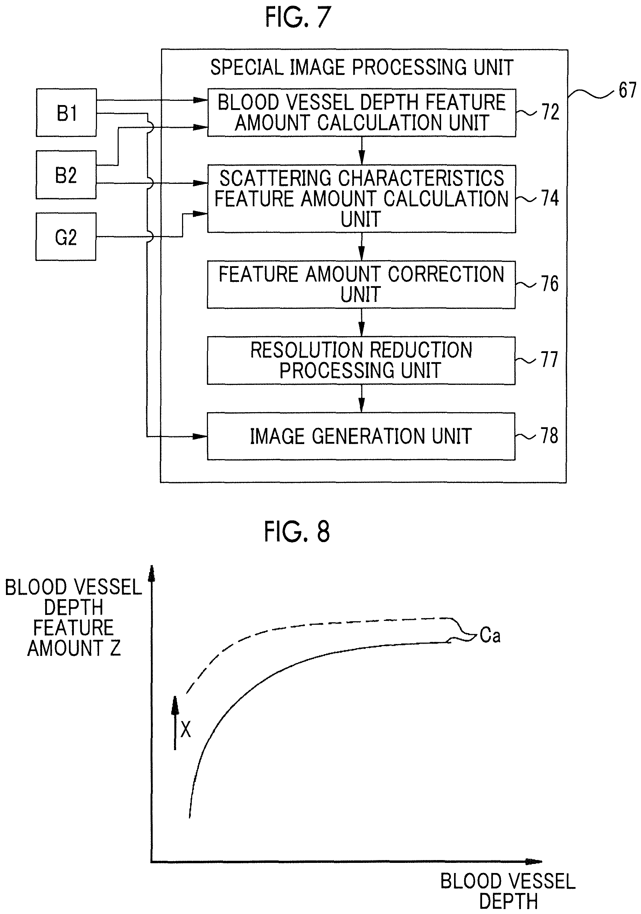

As illustrated in Fig. 7, the special image processing unit 67 includes a blood vessel depth feature amount calculation unit 72, a scattering characteristics feature amount calculation unit 74, a feature amount correction unit 76, a resolution reduction processing unit 77, and an image generation unit 78.

The blood vessel depth feature amount calculation unit 72 performs computation using the B1 image signal and the B2 image signal subject to the alignment processing and the brightness correction processing, and calculates the blood vessel depth feature amount obtained by converting the blood vessel depth in the observation object into an index value. The blood vessel depth indicates a distance in a depth direction from a mucous membrane surface to a blood vessel at a specific position. The blood vessel depth feature amount calculation unit 72 is capable of calculating the depth of a blood vessel located in an extremely shallow layer of an observation tissue, for example, a blood vessel depth of 200 $\mu$m or less is possible from the mucous membrane surface.

In the blood vessel depth feature amount calculation unit 72, specifically, the difference or ratio of the B1 image signal and the B2 image signal is calculated. In the present embodiment, the blood vessel depth feature amount calculation unit 72 log-transforms the B1 image signal and the B2 image signal, and generates a difference between the B1 image signal and the B2 image signal after the logarithmic transformation, more specifically, and a blood vessel depth feature amount Z $(=\ln(B1/B2))$ obtained by subtracting the B2 image signal from the B1 image signal. In a case where the B1 image signal and the B2 image signal are used as they are without being log transformed, the blood vessel depth feature amount Z is generated by computing the ratio of the B1 image signal and the B2 image signal for each pixel.

In addition, as a reason to log-transform, the B1 image signal and the B2 image signal have pixel values proportional to densities in a case where these signals are log transformed, although respective pixels have pixel values proportional to received light quantities. Thus, stable computation results can be obtained irrespective of the brightness of illumination light in a case where respective image signals are obtained.

Regarding the blood vessel depth feature amount, for example, in a case where the B2 image signal is subtracted from the B1 image signal after the logarithmic transformation, particularly, the blood vessel depth feature amount Z of the pixel indicating extreme surface layer blood vessels at a shallow depth position As under a mucous membrane becomes a small value. On the contrary, the blood vessel depth feature amount Z of pixels indicating the surface layer blood vessels at a deep depth position Ad deeper than the extreme surface layer blood vessels becomes a large value. In this way, since the value of the blood vessel depth feature amount varies depending on a difference in blood vessel depth, the blood vessel depth feature amount Z can be used as an index indicating the blood vessel depth.

Here, in a case where a two-dimensional space in which a vertical axis is the blood vessel depth feature amount Z and the horizontal axis is the blood vessel depth is plotted, as illustrated in Fig. 8, the blood vessel feature amount Z of blood vessels at a shallow position like extreme surface layer blood vessels among blood vessels is specifically small, and the value of the blood vessel depth feature amount Z become gradually larger in accordance with the blood vessel depth in a case where the blood vessel depth becomes a certain value or more. On the other hand, a characteristic curve Ca indicating a relationship between the blood vessel depth and the blood vessel depth feature amount shifts in a vertical-axis plus direction (X direction) in a case where scattering of light in the observation object becomes strong. As the scattering intensity of light becomes larger, the amount of the shift in the X direction becomes larger. For that reason, in a case where the relationship between the blood vessel depth and the blood vessel depth feature amount varies depending on the scattering intensity of light in the observation object, it is difficult to accurately calculate the blood vessel depth. Thus, in the invention, the scattering characteristics feature amount W obtained by converting the scattering characteristics of the observation object into an index value is calculated, and the blood vessel depth feature amount Z is corrected using the calculated scattering characteristics feature amount W such that the influence of light scattering of the observation object is eliminated.

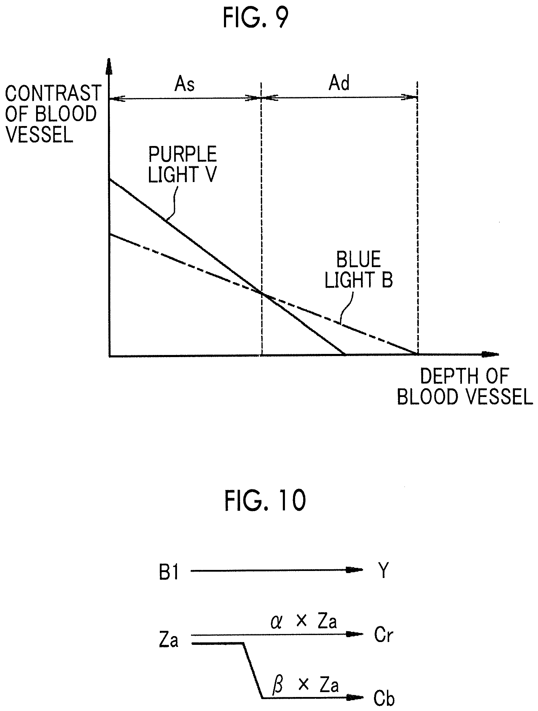

In addition, the reason why the blood vessel depth feature amount varies depending on the blood vessel depth is as follows. For example, as illustrated in Fig. 9, in a case where the purple light V used for the acquisition of the B1 image signal and the blue light B used for the acquisition of the B2 image signal are used as the illumination light, any blood vessel is observable because blood vessels in a total range of the shallow depth position As and the deep depth position Ad, such as the extreme surface layer blood vessels and the surface layer blood vessels, have a constant blood vessel contrast. Here, the "blood vessel contrast" means the ratio of the quantity of reflected light from a surrounding mucous membrane to the quantity of reflected light from the blood vessels.

Since the purple light V has a wavelength shorter than the blue light B, the degree of reach to the observation object is small, and only blood vessels present at the relatively shallow depth position As under the mucous membrane with respect to the blue light B are projected, whereas the contrast of the blood vessels present at the shallow depth position As is larger than that in a case where the blue light B is used. Meanwhile, since the blue light B has a wavelength longer than the purple light V, the degree of reach to the observation object is large, and only blood vessels present at the relatively deep depth position Ad under the mucous membrane with respect to the purple light V are imaged, whereas the contrast of the blood vessels present in at the shallow depth position As is smaller than that in a case where the purple light V is used. The difference in the blood vessel contrast resulting from the difference in the depth of blood vessels as above being represented by the difference between the B1 image signal obtained from the purple light V and the B2 image signal obtained from the blue light B becomes the blood vessel depth feature amount Z.

The scattering characteristics feature amount calculation unit 74 calculates the scattering characteristics feature amount W used for the correction of the blood vessel depth feature amount Z calculated by the blood vessel depth feature amount calculation unit 72. The scattering characteristics feature amount W is a feature amount on which the scattering characteristics of the observation object is reflected. Since the scattering characteristics feature amount W does not include the blood vessel depth feature amount Z, the scattering characteristics feature amount W is not influenced by the blood vessel depth. Since the scattering characteristics feature amount W does not include an oxygen saturation feature amount, either, the scattering characteristics feature amount W is not influenced by oxygen saturation. In order to acquire such a scattering characteristics feature amount W, it is preferable to use at least a specific image in a range where the scattering coefficient is relatively high, that is, a specific image having a small change in the blood vessel contrast resulting from the blood vessel depth or oxygen saturation. Specifically, it is preferable to calculate the scattering characteristics feature amount W, using a specific image including wavelength information on a first specific wavelength range of 430 to520 nm.

The first specific wavelength range of 430 to 520 nm is a wavelength range (refer to Fig. 5) having a higher scattering coefficient as compared to other ranges. Additionally, a wavelength range that of 450 to 500 nm a small change in the blood vessel contrast resulting from the blood vessel depth is also included in the specific wavelength range. Moreover, equal absorption wavelengths of 450 nm and 500 nm at which the light absorption coefficient of oxygenated hemoglobin HbO2 and the light absorption coefficient of reduced hemoglobin Hb become equal to each other are included in the specific wavelength range.

Image signals including the wavelength information on the first specific wavelength range of 430 to 520 nm are equivalent to the B2 image signal and the G2 image signal. However, as illustrated in the spectrum distribution of the blue light B (the blue light B after transmission through a wavelength cutoff filter) and the green light G used for the acquisition of the image signals (refer to Fig. 3), the wavelength information of both the B2 image signal and the G2 image signal near 460 to 500 nm decreases as compared to wavelength information in other ranges. Shortage of the wavelength information near 460 to 500 nm improves the blood vessel contrast. As a result, the resolving power of the blood vessel depth, that is, the blood vessel depth feature amount Z, is included in the B2 image signal and the G2 image signal.

Thus, in the first embodiment, first, as shown below, the scattering characteristics feature amount calculation unit 74 generates a specific image including wavelength information near 460 to 500 nm by performing weighted addition on the B2 image signal and the G2 image signal (Equation 1).

$$(\text{Equation 1}) \qquad (a \times B2 + (1 - a) \times G2) \qquad (= \text{Specific Image})$$

Here, "a" of (Equation 1) is a weighting coefficient, and is in a range of 0 < a < 1. "B2" of (Equation 1) indicates a pixel value of the B2 image signal, and "G2" represents a pixel value of G2 image signal. In order to reliably include the wavelength information near 460 to 500 nm, it is preferable to set the weighting coefficient "a" to a value away from "0" or "1".

Then, as shown below, the scattering characteristics feature amount calculation unit 74 obtains the scattering characteristics feature amount W by logarithmize the specific image after being divided by the G2 image signal (Equation 2). The scattering characteristics feature amount W indicates relative scattering intensity of a substantially short wavelength range, and it becomes large, and becomes larger as the scattering intensity of light in the observation object is larger.

$$(\text{Equation 2}) \qquad \ln \; (\text{Specific Image} \; (a \; \times \; B2 \; + \; (1 \; - \; a) \; \times \; G2)/G2) \; (= \text{Scattering Characteristics Feature Amount W})$$

In addition, the G2 image signal is an image signal obtained from the green light G with a wavelength range, and become an image signal with a small change in the blood vessel contrast resulting from the blood vessel depth or oxygen saturation in a case where the spectrum distribution (refer to Fig. 3) of the green light G is taken into consideration.

The feature amount correction unit 76 obtains the corrected blood vessel depth feature amount Za by correcting the blood vessel depth feature amount Z, using the scattering characteristics feature amount W. Specifically, as shown in the following Equation (3), a value obtained by multiplying the scattering characteristics feature amount W by a certain coefficient b (> 0) is subtracted as a correction amount from the blood vessel depth feature amount Z.

$$(\text{Equation 3}) \qquad Za = Z - b \times W$$

As described above, as the scattering intensity of the observation object becomes larger, the value of the blood vessel depth feature amount Z becomes larger. In contrast, in the feature amount correction unit 76, as the scattering characteristics feature amount W becomes larger, the correction amount to be subtracted from the blood vessel depth feature amount Z becomes larger. Accordingly, since the influence of scattering characteristics is eliminated from the blood vessel depth feature amount Z, the corrected blood vessel depth feature amount Za after correction accurately indicates the blood vessel depth irrespective of the scattering intensity of the observation object.

The resolution reduction processing unit 77 is a so-called low-pass filter (hereinafter referred to as LPF), and reduces the resolution of the corrected blood vessel depth feature amount Za corrected by the feature amount correction unit 76. The intensity of the resolution reduction processing that the resolution reduction processing unit 77 performs on the corrected blood vessel depth feature amount Za is determined by the cut-off frequency of the LPF. The cut-off frequency of the LPF is set in advance, and the resolution of the corrected blood vessel depth feature is reduced to be lower than at least the resolution of an original corrected blood vessel depth feature amount Za.

The image generation unit 78 generates an image having a plurality of output channels, using either the B1 image signal or the B2 image signal received by the special image processing unit 67 and the resolution-reduced corrected blood vessel depth feature amount Za. More specifically, the image generation unit 78 generates an image having a brightness channel Y (a first channel) and two color difference channels Cb and Cr (a second channel and a third channel) related to color differences.

The image generation unit 78 allocating either the B1 image signal or the B2 image signal to the brightness channel Y and allocates the resolution-reduced corrected blood vessel depth feature amount Za to the two color difference channels Cb and Cr, thereby generating a blood vessel depth image in which a traveling pattern of the blood vessels is enhanced in colors corresponding to the blood vessel depth. In the case of the present embodiment, the reason why the B1 image signal is allocated to the brightness channel Y is that the extreme surface layer blood vessels are selectively enhanced from the surface layer blood vessels. As illustrated in Fig. 10, the B1 image signal which corresponds to light (purple light V) of a relatively short wavelength range out of the B1 image signal and the B2 image signal and in which the contrast of the extreme surface layer blood vessels is high is allocated to the brightness channel Y. Also, the corrected blood vessel depth feature amount Za is allocated to the color difference channels Cb and Cr. Additionally, in a case where the corrected blood vessel depth feature amount Za is allocated to the color difference channels Cb and Cr, multiplication is made by a coefficient $\alpha$ and a coefficient $\beta$, respectively. This is for aligning an image and tone to be displayed by an endoscopic system that enhances and observes the surface layer blood vessels or the like.

Specifically, in related-arts endoscopic system having an enhancement observation mode of enhancing and observing surface layer blood vessels, in the case of an enhancement observation mode, narrow-band blue light is radiated to image an observation object to acquire a B image signal, and narrow-band green light is radiated to image the observation object to acquire a G image signal. Then, by allocating the B image signal to a B channel and a G channel of a display image and allocating the G image signal to an R channel, the middle-depth blood vessels at the deep position under the mucous membrane are turned into a green-based (cyan-based) color, and the surface layer blood vessels at the shallow position under the mucous membrane are turned into a red-based (magenta-based) color and are enhanced and displayed. In ITU-R.601, a relationship between the respective RGB image signals, the brightness channel Y, and the color difference channels Cb and Cr is expressed by the following Equation (K), (L), and (M).

$$\text{(Equation K)} \qquad Y = 0.299R + 0.587G + 0.114B$$

$$\text{(Equation L)} \qquad Cb = -0.169R - 0.331G + 0.5B$$

$$\text{(Equation M)} \qquad Cr = 0.5R - 0.419G - 0.081B$$

Then, in Equation (L) and Equation (M) of the color difference channels Cb and Cr, in a case where G is substituted for R and B is substituted for G, the color difference channels Cb and Cr can be expressed with (G-B) as shown in Equation (P) and Equation (Q).

$$\text{(P)} \qquad Cb = -0.169G + 0.169B = 0.169 \, (G - B)$$

$$\text{(Q)} \qquad Cr = 0.5G - 0.5B = 0.5 \, (G - B)$$

In the present embodiment, since the extreme surface layer blood vessels are extracted and displayed, the corrected blood vessel depth feature amount Za is used instead of this (G-B) signal. That is, multiplication by a coefficient $\alpha = 0.169$ to allocate the corrected blood vessel depth feature amount Za to a color-difference signal Cb, and multiplication is made by the coefficient $\beta = 0.5$ to allocate the corrected blood vessel depth feature amount Za to a color-difference signal Cr. Accordingly, an image of substantially the same color scheme as the related-art endoscopic systems is displayed in the endoscopic system 10. Here, in order to enhance differences in color between the extreme surface layer blood vessels and the surface layer blood vessels at the relatively deep position, there is a case where the above coefficient $\alpha$ and the above coefficient $\beta$ may be further multiplied by coefficients in accordance with settings or the like.

In addition, in order to generate the blood vessel depth image of RGB from the brightness channel Y and the color difference channels Cb and Cr, the followings are performed in accordance with the inverse transformation of ITU-R.601.

$$\text{(Equation S)} \qquad R = Y + 1.402Cr$$

$$\text{(Equation T)} \qquad G = Y - 0.344Cb - 0.714Cr$$

$$\text{(Equation U)} \qquad B = Y + 1.772Cb$$

The normal image generated by the normal image processing unit 66, and the blood vessel depth image generated by the special image processing unit 67 are input to the video signal generation unit 68. The video signal generation unit 68 converts the normal image and the blood vessel depth image into video signals for display as an image that can be displayed by the monitor 18. The monitor 18 displays the normal image and the blood vessel depth image using the video signals.

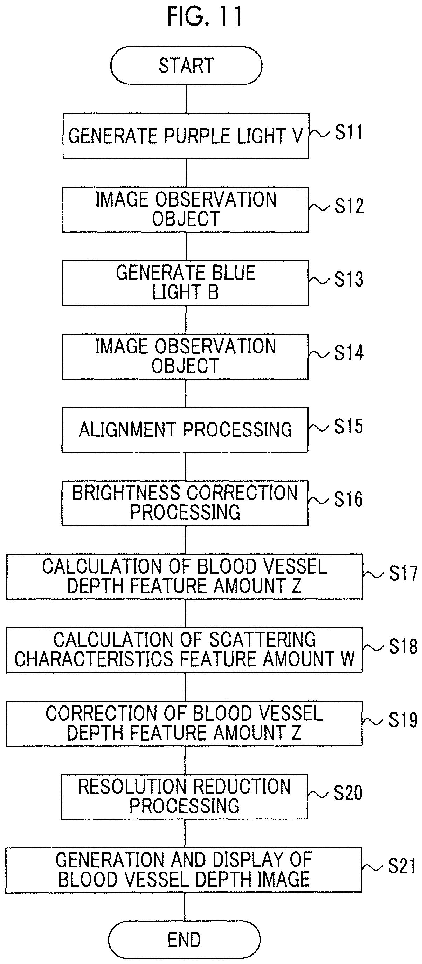

Next, a series of flow of the image processing in the special observation mode will be described with reference to Fig. 11. First, the light source 20 generates the purple light V in the first light emission pattern and irradiates the observation object with the generated purple light V (S11). The imaging sensor 48 images the observation object irradiated with the purple light V (S12), and the image signal acquisition unit 53 acquires the B1 image signal, the G1 image signal, and the R1 image signal.

Next, the B1 light source 20 generates mixed color light of the blue light B, the green light G, and the red light R in the second light emission pattern, and irradiates the observation object with the generated mixed color light of the blue light B, the green light G, and the red light R (S13). The imaging sensor 48 images the observation object irradiated with the blue light B (S14). Then, the image signal acquisition unit 53 acquires the B2 image signal, the G2 image signal, and the R2 image signal.

The image signal and the B2 image signal, which have been obtained as described above, are input to the special image processing unit 67 after being aligned with each other in the alignment processing unit 62 (S15) and further subjected to the brightness correction processing by the brightness correction processing unit 63 (S16). In the special image processing unit 67, the blood vessel depth feature amount calculation unit 72 calculates the blood vessel depth feature amount Z, using the B1 image signal and the B2 image signal (S17). The calculated blood vessel depth feature amount Z becomes a value that is influenced by the scattering characteristics and is different from an actual blood vessel depth, in a case where the scattering intensity of the observation object is strong.

Subsequently, the scattering characteristics feature amount calculation unit 74 calculates the scattering characteristics feature amount W, using the B2 image signal and the G2 image signal (S18). The scattering characteristics feature amount W is used for the correction for eliminating the influence of the scattering characteristics from the blood vessel depth feature amount Z, and does not include the blood vessel depth feature amount or the oxygen saturation feature amount. Then, the feature amount correction unit 76 corrects the blood vessel depth feature amount Z, using the scattering characteristics feature amount W (S19). Accordingly, the influence of the scattering characteristics of the observation object is eliminated, and the corrected blood vessel depth feature amount Za accurately indicating the blood vessel depth is obtained.

After the corrected blood vessel depth feature amount Za is obtained, the corrected blood vessel depth feature amount Za is further resolution-reduced by the resolution reduction processing unit 77 (S20). Thereafter, the image generation unit 78 generates the blood vessel depth image by allocating the B1 image signal to a brightness channel Y to allocate the resolution-reduced corrected blood vessel depth feature amount Za to the color difference channels Cr and Cb. The generated blood vessel depth image is displayed on the monitor 18 (S21).

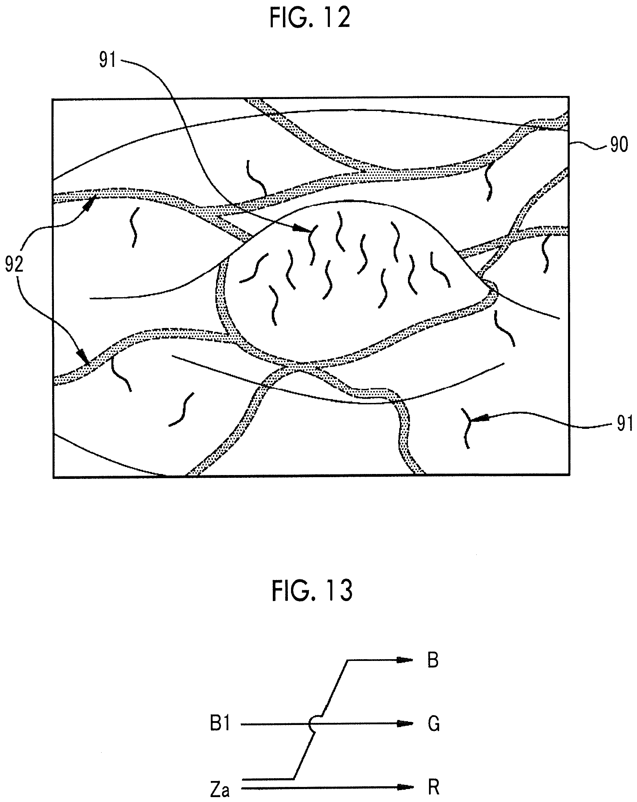

Specifically, as illustrated in Fig. 12, in the blood vessel depth image 90, the extreme surface layer blood vessels 91 are colored in a magenta-based color and displayed, and the surface layer blood vessels 92 at the position deeper than the extreme surface layer blood vessels is colored in a cyan-based color and displayed. For this reason, in the blood vessel depth image 90 the extreme surface layer blood vessels 91 and the surface layer blood vessels 92 are capable of being discriminated from each other in color, and the extreme surface layer blood vessels 91 are displayed more brightly than the surface layer blood vessels 92. Therefore, the extreme surface layer blood vessels 91 are displayed in a state where the extreme surface layer blood vessels are easy to observe.

In addition, in the above embodiment, the image generation unit 78 allocates the B1 image signal with a relatively high contrast of the extreme surface layer blood vessels out of the B1 image signal and the B2 image signal to the brightness channel Y, and allocates the corrected blood vessel depth feature amount Za to the color difference channels Cb and Cr, thereby generating the blood vessel depth image in which the extreme surface layer blood vessels 91 are selectively enhanced. However, the image generation unit 78 may generate a specific depth blood vessel image in which the surface layer blood vessels 92 at the relatively deep position are enhanced.

In this case, the blood vessel depth feature amount calculation unit 72 subtracts the B2 image signal from the B1 image signal after the logarithmic transformation, to generate the blood vessel depth feature amount Z, contrary to the above embodiment. The corrected blood vessel depth feature amount Za is obtained by correcting the blood vessel depth feature amount Z in the feature amount correction unit 76. Then, the image generation unit 78 generates the blood vessel depth image by allocating the B2 image signal to the brightness channel Y to allocate the corrected blood vessel depth feature amount Za to the color difference channels Cb and Cr.

In a case where the image generation unit 78 generates the blood vessel depth image, it is preferable to select which of the B1 image signal and the B2 image signal is to be allocated to the brightness channel Y. For example, a first mode where the B1 image signal is allocated to the brightness channel Y, and a second mode where the B2 image signal is allocated to the brightness channel Y is prepared as operation modes of the image generation unit 78, and an image can be generated in a mode selected out of the first mode and the second mode.

Additionally, in a case where it is possible to select an image signal to be allocated to the brightness channel Y, the image generation unit 78 may automatically select the image signal to be allocated to the brightness channel Y. For example, the B1 image signal may be compared with the B2 image signal, and both the image signals or an image signal with less noise within a specified region of interest may be automatically allocated to the brightness channel Y, or both the image signals or an image signal with a higher contrast within the specified region of interest may be automatically allocated to the brightness channel Y.

Additionally, in the above embodiment, the image generation unit 78 allocates the B1 image signal to the brightness channel Y, and allocates the corrected blood vessel depth feature amount Za to the color difference channels Cb and Cr, thereby generating a YCbCr type blood vessel depth image. However, an RGB type image having a red output channel (hereinafter referred to as an R channel), a green output channel (hereinafter referred to as a G channel), and a blue output channel (hereinafter referred to as a B channel) may be generated. In this case, as illustrated in Fig. 13, the image generation unit 78 allocates the B1 image signal to the G channel (first channel) that most contributes to brightness, and allocates the corrected blood vessel depth feature amount Za to the remaining B channel (second channel) and R channel (third channel).

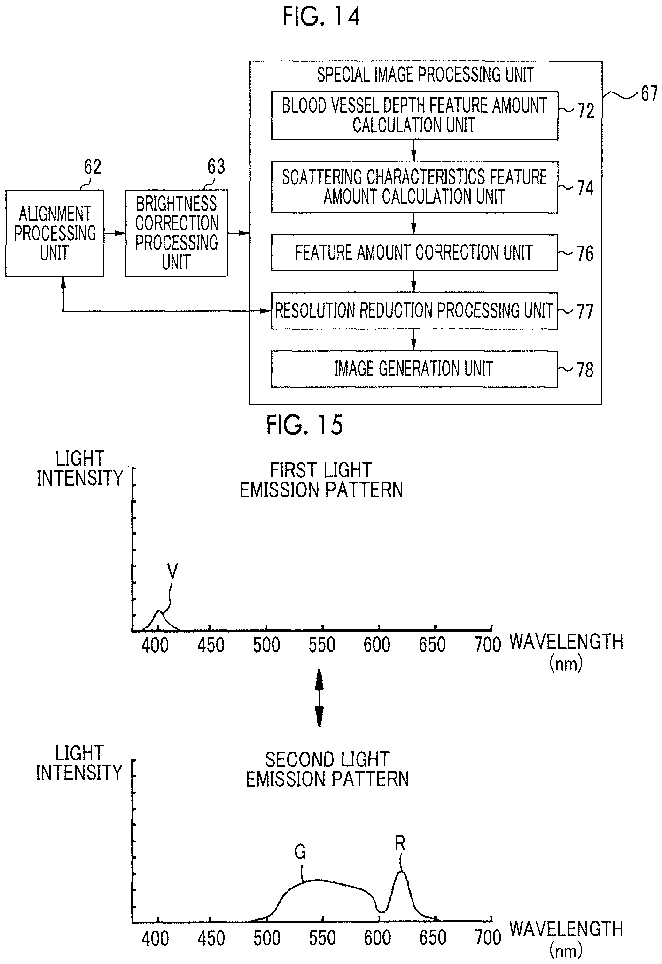

In the above embodiment, the cut-off frequency of the LPF to be used in the resolution reduction processing unit 77 is set in advance. However, it is preferable to make the cut-off frequency of the LPF variable and dynamically set the cut-off frequency of the LPF. For example, as illustrated in Fig. 14, the alignment accuracy of the B1 image signal and the B2 image signal is input from the alignment processing unit 62 to the resolution reduction processing unit 77. Then, the resolution reduction processing unit 77 changes the cut-off frequency (the intensity of the resolution reduction processing) of the LPF in accordance with the alignment accuracy of the B1 image signal and the B2 image signal.

Specifically, as the alignment accuracy of the B1 image signal and the B2 image signal is higher, the cut-off frequency of the LPF may be set to a higher frequency to make the intensity of the resolution reduction processing smaller, and as the alignment accuracy of the B1 image signal and B2 is lower, the cut-off frequency of the LPF may be set to a lower frequency to make the intensity of the resolution reduction processing larger. By doing in this way, the degree of reduction of resolution of the corrected blood vessel depth feature amount Za by the resolution reduction processing unit 77 can be optimized, and the blood vessels (for example, the extreme surface layer blood vessels) at the specific depth can be appropriately enhanced and displayed.

In addition, in a case where the blood vessel depth image is displayed or saved as a still image, it is preferable the cut-off frequency of the LPF is set to be at least within a range where at least a frequency of 1/8 or less of the Nyquist frequency is left, with the resolution of the blood vessel depth image to be generated as a reference.

In the above modification example, the resolution reduction processing unit 77 regulates the intensity of the resolution reduction processing in accordance with the accuracy of alignment processing of the alignment processing unit 62. However, contrary to this, the alignment processing unit 62 may regulate the accuracy of alignment processing in accordance with the intensity of the resolution reduction processing performed by the resolution reduction processing unit 77. In this case, the alignment processing unit 62 set the alignment accuracy of the B1 image signal and the B2 image signal to a higher value as the cut-off frequency of the LPF is set to be larger and the intensity of the resolution reduction processing is set to be smaller.

in a case where the accuracy of alignment processing of the B1 image signal and the B2 image signal performed by the alignment processing unit 62 is made variable and the still image of the blood vessel depth image is displayed or saved, and in a case where a moving image of the blood vessel depth image is displayed, it is preferable to change the accuracy of alignment processing. For example, in a case where the moving image constituted of the specific depth blood vessel image is displayed on the monitor 18, the alignment processing unit 62 aligns the B1 image signal and the B2 image signal with each other with a first accuracy lower than that in a case where the still image of the specific depth blood vessel image is displayed (or saved) on the monitor 18.

Contrary to this, in a case where the still image of the specific depth blood vessel image is displayed on the monitor 18, the alignment processing unit 62 aligns the B1 image signal and the B2 image signal with each other with a second accuracy higher than that in a case where the moving image of the specific depth blood vessel image is displayed on the monitor 18. By doing in this way, at the time of the display of the moving image, the blood vessel depth image can be generated at high speed within a range where the color deviation is not conspicuous, and at the time of the acquisition of a still image with a conspicuous color deviation, the blood vessel depth image without a color deviation can be generated.

Additionally, the alignment processing unit 62 may change the alignment accuracy of the B1 image signal and the B2 image signal depending on the size of a blood vessel depth image to be generated. For example, in a case where the blood vessel depth image to be generated is large, a slight positional deviation is also conspicuous. Thus, the alignment processing unit 62 performs the alignment of the B1 image signal and the B2 image signal with high accuracy, in a case where the blood vessel depth image to be generated is small, a slight positional deviation is also conspicuous. Thus, the alignment of the B1 image signal and the B2 image signal is performed with low accuracy.

Contrary to this, the alignment processing unit 62 may perform the alignment of the B1 image signal and the B2 image signal with low accuracy in a case where the blood vessel depth image to be generated is large, and may perform the alignment of the B1 image signal and the B2 image signal with high accuracy in a case where the blood vessel depth image to be generated is small. By doing in this way, a processing burden on the processor device 16 can be optimized.

As described above, in a case where the alignment processing unit 62 changes the accuracy of alignment processing at the time of the display of the moving image and the acquisition of the still image or in a case where the alignment processing unit 62 changes the alignment accuracy in accordance with to the size of the blood vessel depth image, it is preferable that the resolution reduction processing unit 77 changes the cut-off frequency of the LPF depending on the alignment accuracy. For example, at the time of the display of the moving image, the alignment processing unit 62 may lower the alignment accuracy of the B1 image signal and the B2 image signal, and instead this, the cut-off frequency of the LPF may be shifted to a low-frequency side in the resolution reduction processing unit 77.

Additionally, at the time of the acquisition of the still image, the alignment processing unit 62 may raise the alignment accuracy of the B 1 image signal and the B2 image signal, and instead of this, the cut-off frequency of the LPF may be shifted to a high-frequency side in the resolution reduction processing unit 77. That is, at the time of the display of the moving image, a priority may be given to the LPF of the resolution reduction processing unit 77 in which the processing burden on the processor device 16 is small, and at the time of the acquisition of the still image, a priority may be given to the accurate alignment by the alignment processing unit 62. In addition, the alignment processing unit 62 may not perform the alignment of the B1 image signal and the B2 image signal at the time of the display of the moving image, and may perform the alignment of the B1 image signal and the B2 image signal only at the time of the acquisition of the still image.

In the above embodiment, although the resolution reduction processing unit 77 reduces the resolution of the corrected blood vessel depth feature amount Za by the LPF, the resolution can also be reduced by reducing the corrected blood vessel depth feature amount Za and then enlarging the corrected blood vessel depth feature amount Za up to its original size instead of the LPF. In this way, in a case where the corrected blood vessel depth feature amount Za is reduced and enlarged to reduce the resolution, it is preferable to adopt a reduction method with less aliasing at the time of reduction of the corrected blood vessel depth feature amount Za. For example, the corrected blood vessel depth feature amount Za can be reduced in resolution after being reduced by the area average method and then enlarged by cubic spline interpolation.

As in the above embodiment, in a case where blood vessels distributed on a tissue surface layer, such as the extreme surface layer blood vessels and the surface layer blood vessels, are distinguished from each other and displayed in an enhanced manner depending on the blood vessel depth, it is preferable that both wavelength ranges of two-wavelengths of illumination light to be used are within a wavelength range of 500 nm or less like the purple light V and the blue light B. On the other hand, in a case where blood vessels distributed on a tissue middle layer or a tissue deep layer are distinguished from each other and displayed in an enhanced manner depending on the blood vessel depth, it is preferable that both wavelength ranges of two-wavelengths of illumination light to be used are 500 nm or more.

[Second embodiment]

In the first embodiment, in the special observation mode, in order to obtain the image signals required for the calculation and correction of the blood vessel depth feature amount Z, the purple light V is emitted in the first light emission pattern, and the mixed color light of the blue light B, the green light G, and the red light R is emitted in the second light emission pattern. However, in a second embodiment, as illustrated in Fig. 15, the purple light V is emitted in the first light emission pattern similarly to the first embodiment, while in the second light emission pattern, the blue light B is not emitted and the green light G and the red light R are emitted.

Hence, the wavelength information corresponding to the purple light V is included in the B1 image signal among the image signals obtained by irradiating the observation object with the purple light V in the first light emission pattern to image the observation object, similarly to the first embodiment. In contrast, the B2 image signal among the image signals obtained by irradiating the observation object with the green light G and the red light R in the second light emission pattern to image the observation object includes only the wavelength information corresponding to the light of 480 to 560 nm of the green light G, and G2 image signal includes only the wavelength information corresponding to the green light G. Meanwhile, the R2 image signal is the same as that of the first embodiment.

As described above, since only the wavelength information corresponding to the light of 480 to 560 nm is included in the B2 image signal, the information of the surface layer blood vessels decreases unlike the first embodiment as compared to the B2 image signal also including the wavelength information corresponding to the blue light B. Additionally, the B2 image signal is also not easily influenced by the oxygen saturation. Similarly to the B2 image signal, the G2 image signal has little information of the surface layer blood vessels and is not easily influenced by the oxygen saturation.

In the second embodiment, similarly to the first embodiment, the blood vessel depth feature amount calculation unit 72 calculates the blood vessel depth feature amount Z, using the B1 image signal and the B2 image signal. As described above, the B2 image signal used for the calculation of the blood vessel depth feature amount Z has little information of the surface layer blood vessels. Therefore, the blood vessel depth feature amount Z has almost no information of the extreme surface layer blood vessels at a shallow position than the surface layer blood vessels. Then, similarly to the first embodiment, the scattering characteristics feature amount calculation unit 74 calculates the scattering characteristics feature amount W, using the B2 image signal and the G2 image signal. The scattering characteristics feature amount W is obtained by dividing the specific image obtained by performing weighted addition of the B2 image signal and the G2 image signal by the G2 image signal as shown above (Equation 2). Then, the feature amount correction unit 76 corrects the blood vessel depth feature amount Z, using the scattering characteristics feature amount W, by the same method as that in the first embodiment. Accordingly, the corrected blood vessel depth feature amount Za is obtained.

In addition, it is preferable that the scattering characteristics feature amount W includes the wavelength information in the range where the scattering coefficient is relatively high as described above, and include the wavelength information in the second specific wavelength range of 480 to 520 nm in order to reduce a change in the blood vessel contrast resulting from the blood vessel depth or the oxygen saturation feature amount. In the second embodiment, in the case of weighted addition of the B2 image signal and the G2 image signal, it is preferable to bring the weighting coefficient "a" close to "1" in order to increase weighting of the B2 image signal including the wavelength information corresponding to the light in the second specific wavelength range of 480 to 520 nm in the green light G.

[Third embodiment]

In the first embodiment, the specific image in the specific wavelength range of 430 520 nm, including the wavelength information in the range where the scattering coefficient is relatively high and having a small change in the blood vessel contrast resulting from the blood vessel depth or the oxygen saturation, is generated by the weighted addition of the B2 image signal and the G2 image signal. However, in the third embodiment, a specific image is generated without performing the weighted addition of the B2 image signal and the G2 image signal by emitting blue-green light Bx having a wavelength range between the blue light B and the green light B.

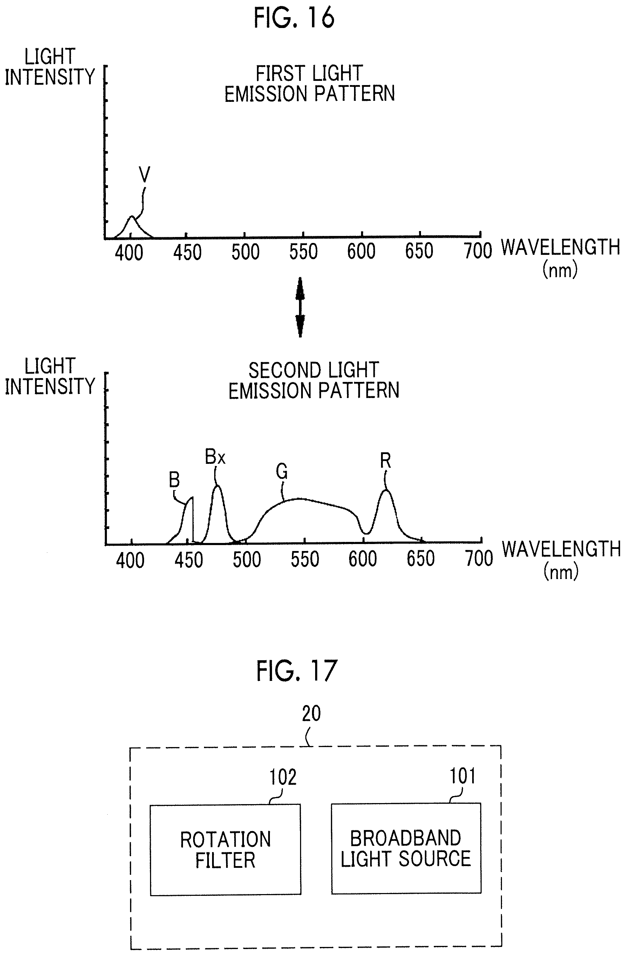

In the third embodiment, as illustrated in Fig. 16, the purple light V is emitted is emitted in the first light emission pattern similarly to the first embodiment, while in the second light emission pattern, the blue-green light Bx with a central wavelength of 470 ± 10 nm is emitted in addition to the mixed color light of the blue light B, the green light G, and the red light R. The blue-green light Bx may be emitted from an LED or may be emitted by an LD (Laser Diode), similarly to the blue light B.

Hence, the B1 image signal among the image signals obtained by irradiating the observation object with the purple light V in the first light emission pattern to image the observation object includes the wavelength information corresponding to the purple light V, similarly to the first embodiment. In contrast, the B2 image signal among the image signals obtained by irradiating the observation object with the blue light B, the blue-green light Bx, the green light G, and the red light R in the second light emission pattern to image the observation object also include the wavelength information corresponding to the blue-green light Bx, in addition to the wavelength information corresponding to the blue light B and the wavelength information corresponding to the light of 480 to 560 nm of the green light G. The G2 image signal also includes the wavelength information corresponding to the blue-green light Bx in addition to the wavelength information corresponding to the light up to 450 to 500 nm and the wavelength information corresponding to the green light G, in the blue light B. Meanwhile, the R2 image signal is the same as that of the first embodiment.

Then, in the third embodiment, the scattering characteristics feature amount calculation unit 74 calculates the scattering characteristics feature amount W by dividing the specific image including the B2 image signal by the G2 image signal. In addition, the calculation and correction of the blood vessel depth feature amount Z other than the calculation of the scattering characteristics feature amount W is the same as that of the first embodiment.

[Fourth embodiment]

In the above first to third embodiments, light emission is performed using the semiconductor light sources in a plurality of colors. However, in the fourth embodiment, light emission in a plurality of colors is performed using a broadband light source, such as a xenon lamp, and a rotation filter. In the fourth embodiment, as illustrated in Fig. 17, the light source 20 includes a broadband light source 101 and a rotation filter 102. The broadband light source 101 is a xenon lamp, a halogen lamp, a white LED, or the like, and generates white broadband light ranging from a blue range to a red range. The rotation filter 102 is rotatably disposed on an optical path of broadband light emitted from the broadband light source 101, and limits a wavelength range of the broadband light to make the broadband light incident on the light guide 41.

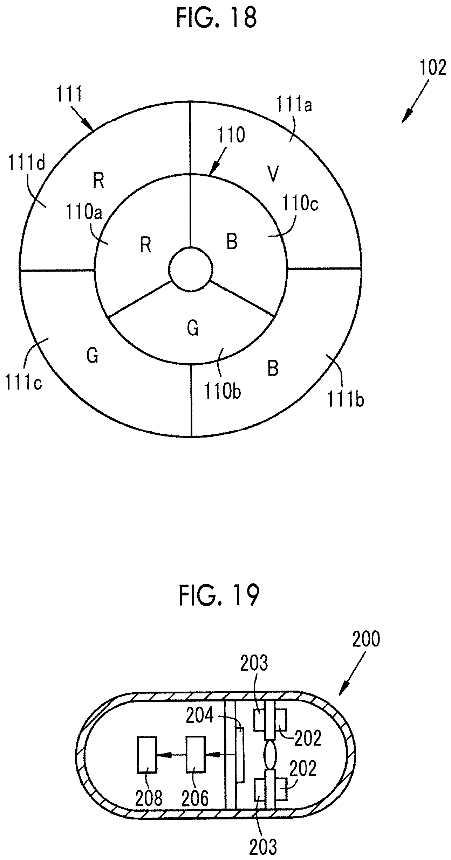

As illustrated in Fig. 18, the rotation filter 102 has a normal observation mode filter 110 and a special observation mode filter 111. The normal observation mode filter 110 has a red filter (R) 110a that transmits the red light R, a green filter (G) 110b that transmits the green light G, and a blue filter (B) 110c that transmits the blue light B. It is preferable that the red light R, the green light G, and the blue light B that transmit the filters 110a to 110c are the same in wavelength range as the red light R, the green light G, and the blue light B illustrated in the above first to third embodiments.

The special observation mode filter 111 has a purple filter (V) 111a that transmits the purple light, a blue filter (B) 111b that transmits the blue light, a green filter (G) 111c that transmits the green light (G), and a red filter (R) 111d that transmits the red light R. In the rotation filter 102, the normal observation mode filter 110 or the special observation mode filter 111 is disposed on the optical path of the broadband light in accordance with a set mode.

Additionally, the rotation filter 102 rotates in synchronization with the imaging frame of the imaging sensor 48 in accordance with the set mode. In a case where the normal observation mode is set, imaging is performed whenever the red light R, the green light G, and the blue light B are sequentially radiated in synchronization with the rotation of the rotation filter 102 and light of each color is radiated. Here, an image signal obtained at the time of the radiation of the red light R corresponds to the Rc image signal, an image signal obtained at the time of the radiation of the green light G corresponds to the Gc image signal, and an image signal obtained at the time of the radiation of the blue light B corresponds to the Bc image signal.

Meanwhile, in a case where the special observation mode is set, imaging is performed whenever the purple light V, the blue light B, the green light G, and the red light R are sequentially radiated in synchronization with the rotation of the rotation filter 102 and light of each color is radiated. Here, an image signal obtained at the time of the radiation of the purple light V corresponds to the B1 image signal. An image signal obtained at the time of the radiation of the blue light B substantially corresponds to the B2 image signal, an image signal obtained at the time of the radiation of the green light G substantially corresponds to the G2 image signal, and an image signal obtained at the time of the radiation of the red light R substantially corresponds to the R2 image signal. In addition, in the fourth embodiment, a monochrome imaging sensor may be used as the imaging sensor 48 instead of the color imaging sensor.

[Fifth embodiment]

In addition, in the above first to fourth embodiments, the endoscope 12 is inserted into a subject and the illumination and imaging within the subject are performed. However, in the fifth embodiment, as illustrated in Fig. 19, the illumination and imaging within the subject may be performed using a capsule endoscope 200 to be used after being swallowed by a subject from his/her mouth.

The capsule endoscope 200 includes a light source 202, a light source control unit 203, an imaging sensor 204, a signal processing unit 206, and a transmission/reception antenna 208. The light source 202 is configured similarly to the light source 20 of the above embodiment. The light source control unit 203 controls driving of the light source 202, similarly to the light source control unit 22. The imaging sensor 204 images the observation object in accordance with the driving of the light source 202. Image signals obtained by the imaging are sent to a signal processing unit 206.

Additionally, the capsule endoscope 200 is wirelessly connected to the capsule endoscope processor device (not illustrated) by a transmission/reception antenna 208. The capsule endoscope processor device is almost the same as that of the processor device 16 except having a receiving unit (not illustrated) that receives signals from the capsule endoscope 200. Image signals received by the signal processing unit 206 are transmitted to the processor device via the transmission/reception antenna 208.

Explanation of References

| | |
|---|---|
| 10: | endoscopic system |
| 12: | endoscope |
| 12a: | insertion part |
| 12b: | operating part |
| 12c: | bending part |
| 12d: | distal end part |
| 12e: | angle knob |
| 13a: | switch |
| 13b: | zooming operating part |
| 14: | light source device |
| 16: | processor device |
| 18: | monitor |
| 19: | console |
| 20: | light source |
| 22: | light source control unit |
| 23a: | V-LED |
| 23b: | B-LED |
| 23c: | G-LED |
| 23d: | R-LED |
| 24: | wavelength cutoff filter |
| 30a: | illumination optical system |
| 30b: | imaging optical system |

| 41: | light guide |
| --- | --- |
| 45: | illumination lens |
| 46: | objective lens |
| 47: | zoom lens |
| 48: | imaging sensor |
| 51: | circuit |
| 52: | A/D converter |
| 53: | image signal acquisition unit |
| 56: | DSP |
| 58: | noise removal unit |
| 61: | image processing switching unit |
| 62: | alignment processing unit |
| 63: | brightness correction processing unit |
| 66: | normal image processing unit |
| 67: | special image processing unit |
| 68: | video signal generation unit |
| 72: | feature amount calculation unit |
| 74: | scattering characteristics feature amount calculation unit |
| 76: | feature amount correction unit |
| 77: | resolution reduction processing unit |
| 78: | image generation unit |
| 90: | blood vessel depth image |
| 91: | extreme surface layer blood vessel |
| 92: | surface layer blood vessel |
| 101: | broadband light source |
| 102: | rotation filter |
| 110: | normal observation mode filter |
| 110a: | red filter (R) |
| 110b: | green filter (G) |
| 110c: | blue filter (B) |
| 111: | special observation mode filter |
| 111a: | purple filter (V) |
| 111b: | blue filter (B) |
| 111c: | green filter (G) |
| 111d: | red filter (R) |
| 200: | capsule endoscope |
| 202: | light source |
| 203: | light source control unit |
| 204: | imaging sensor |
| 206: | signal processing unit |
| 208: | transmission/reception antenna |

**Claims**

1. An endoscopic system comprising:

   a blood vessel depth feature amount calculation unit that calculates a blood vessel depth feature amount;
   a scattering characteristics feature amount calculation unit that calculates a scattering characteristics feature amount in which the blood vessel depth feature amount is not included and scattering characteristics in an observation object are reflected;
   a feature amount correction unit that corrects the blood vessel depth feature amount depending on the scattering characteristics feature amount to obtain a corrected blood vessel depth feature amount; and
   a display unit that displays a blood vessel depth image on the basis of the corrected blood vessel depth feature amount.

2. The endoscopic system according to claim 1, further comprising:

an image acquisition unit that acquires a first image, a second image, and a third image that have mutually different wavelength information,

wherein the blood vessel depth feature amount calculation unit calculates the blood vessel depth feature amount by performing computation based on the first image and the second image, and

wherein the scattering characteristics feature amount calculation unit calculates the scattering characteristics feature amount by performing computation based on the second image and the third image.

3. The endoscopic system according to claim 2,
wherein a scattering coefficient of the observation object in a wavelength range of the first image is different from a scattering coefficient of the observation object in a wavelength range of the second image.

4. The endoscopic system according to claim 2 or 3,
wherein the blood vessel depth feature amount is a ratio between the first image and the second image or a difference between the first image and the second image.

5. The endoscopic system according to any one of claims 2 to 4,
wherein the scattering characteristics feature amount calculation unit generates a specific image including wavelength information in a first specific wavelength range of 430 to 520 nm, on the basis of the second image and the third image, and calculates the scattering characteristics feature amount on the basis of the specific image.

6. The endoscopic system according to claim 5,
wherein the second image has wavelength information of a shorter wavelength than the first specific wavelength range, and the third image has wavelength information of a longer wavelength than the first specific wavelength range.

7. The endoscopic system according to claim 5 or 6, further comprising:

a light source that emits at least blue light and green light toward the observation object,
wherein wavelength information corresponding to the blue light is included in the second image, and wavelength information corresponding to the green light is included in the third image.

8. The endoscopic system according to any one of claims 2 to 4,
wherein the scattering characteristics feature amount calculation unit generates a specific image including wavelength information in a second specific wavelength range of 480 to 520 nm, on the basis of the second image and the third image, and calculates the scattering characteristics feature amount on the basis of the specific image.

9. The endoscopic system according to claim 8, further comprising:

a light source that emits at least green light toward the observation object,
wherein wavelength information corresponding to light in the second specific wavelength range in the green light is included in the second image, and wavelength information corresponding to the green light is included in the third image.

10. The endoscopic system according to any one of claims 1 to 9,
wherein an oxygen saturation feature amount is not included in the scattering characteristics feature amount.

11. The endoscopic system according to any one of claims 1 to 10,
wherein the feature amount correction unit obtains the corrected blood vessel depth feature amount by performing a correction in which a correction value obtained by multiplying the scattering characteristics feature amount by a certain coefficient is subtracted from the blood vessel depth feature amount.

12. The endoscopic system according to any one of claims 2 to 9, further comprising:

a resolution reduction processing unit that reduces resolution of the corrected blood vessel depth feature amount; and
an image generation unit that allocates either the first image or the second image to a brightness channel Y and allocates the resolution-reduced corrected blood vessel depth image to color difference channels Cr and Cb to generate the blood vessel depth image.

**13.** An endoscopic system comprising:

an image acquisition unit that acquires a first image, a second image, and a third image that have mutually different wavelength information;

a blood vessel depth feature amount calculation unit that calculates a blood vessel depth feature amount by performing computation based on the first image and the second image;

a scattering characteristics feature amount calculation unit that calculates a scattering characteristics feature amount in which the blood vessel depth feature amount is not included and scattering characteristics in an observation object are reflected, the scattering characteristics feature amount calculation unit calculating the scattering characteristics feature amount by performing computation based on the second image and the third image;

a feature amount correction unit that obtains a corrected blood vessel depth feature amount by performing a correction in which a correction value obtained by multiplying the scattering characteristics feature amount by a certain coefficient is subtracted from the blood vessel depth feature amount;

a resolution reduction processing unit that reduces resolution of the corrected blood vessel depth feature amount;

an image generation unit that allocates either the first image or the second image to a brightness channel Y and allocates the resolution-reduced corrected blood vessel depth image to color difference channels Cr and Cb to generate a blood vessel depth image;

a display unit that displays the blood vessel depth image.

**14.** A processor device comprising:

a blood vessel depth feature amount calculation unit that calculates a blood vessel depth feature amount;

a scattering characteristics feature amount calculation unit that calculates a scattering characteristics feature amount in which the blood vessel depth feature amount is not included and scattering characteristics in an observation object are reflected; and

a feature amount correction unit that corrects the blood vessel depth feature amount depending on the scattering characteristics feature amount to obtain a corrected blood vessel depth feature amount.

**15.** A method of operating an endoscopic system comprising:

a step of calculating a blood vessel depth feature amount, using a blood vessel depth feature amount calculation unit;

a step of calculating a scattering characteristics feature amount in which the blood vessel depth feature amount is not included and scattering characteristics in an observation object are reflected, using a scattering characteristics feature amount calculation unit;

a step of correcting the blood vessel depth feature amount depending on the scattering characteristics feature amount to obtain a corrected blood vessel depth feature amount, using a feature amount correction unit; and

a step of displaying a blood vessel depth image on the basis of the corrected blood vessel depth feature amount, using a display unit.

# FIG. 1

EP 3 520 675 A1

# FIG. 2

# FIG. 3

LIGHT INTENSITY

V   B   Ba   G   R

WAVELENGTH (nm)

400   450   500   550   600   650   700

# FIG. 4

LIGHT INTENSITY

FIRST LIGHT EMISSION PATTERN

V

400   450   500   550   600   650   700   WAVELENGTH (nm)

LIGHT INTENSITY

SECOND LIGHT EMISSION PATTERN

B   G   R

400   450   500   550   600   650   700   WAVELENGTH (nm)

## FIG. 5

SCATTERING COEFFICIENT

PURPLE LIGHT V    BLUE LIGHT B

WAVELENGTH

## FIG. 6

B COLOR FILTER    G COLOR FILTER    R COLOR FILTER

TRANSMITTANCE (%)

100

50

400    500    600    700

WAVELENGTH (nm)

## FIG. 7

SPECIAL IMAGE PROCESSING UNIT                    ～67

B1 → BLOOD VESSEL DEPTH FEATURE
AMOUNT CALCULATION UNIT    ～72

B2 → SCATTERING CHARACTERISTICS
FEATURE AMOUNT CALCULATION
UNIT    ～74

G2 → FEATURE AMOUNT CORRECTION
UNIT    ～76

RESOLUTION REDUCTION
PROCESSING UNIT    ～77

IMAGE GENERATION UNIT    ～78

## FIG. 8

BLOOD VESSEL
DEPTH
FEATURE
AMOUNT Z

Ca

X

BLOOD VESSEL
DEPTH

## FIG. 9

CONTRAST OF BLOOD VESSEL

As

Ad

PURPLE LIGHT V

BLUE LIGHT B

DEPTH OF BLOOD VESSEL

## FIG. 10

B1 $\longrightarrow$ Y

Za $\xrightarrow{\alpha \times Za}$ Cr

$\xrightarrow{\beta \times Za}$ Cb

# FIG. 11

```
        ( START )
            |
  GENERATE PURPLE LIGHT V  ~ S11
            |
   IMAGE OBSERVATION      ~ S12
        OBJECT
            |
    GENERATE BLUE         ~ S13
      LIGHT B
            |
   IMAGE OBSERVATION      ~ S14
        OBJECT
            |
  ALIGNMENT PROCESSING    ~ S15
            |
  BRIGHTNESS CORRECTION   ~ S16
      PROCESSING
            |
  CALCULATION OF BLOOD VESSEL    ~ S17
   DEPTH FEATURE AMOUNT Z
            |
   CALCULATION OF SCATTERING          ~ S18
 CHARACTERISTICS FEATURE AMOUNT W
            |
   CORRECTION OF BLOOD VESSEL    ~ S19
    DEPTH FEATURE AMOUNT Z
            |
  RESOLUTION REDUCTION    ~ S20
      PROCESSING
            |
  GENERATION AND DISPLAY OF     ~ S21
  BLOOD VESSEL DEPTH IMAGE
            |
         ( END )
```

FIG. 12

FIG. 13

## FIG. 14

SPECIAL IMAGE PROCESSING UNIT ~67

BLOOD VESSEL DEPTH FEATURE AMOUNT CALCULATION UNIT ~72

↓

SCATTERING CHARACTERISTICS FEATURE AMOUNT CALCULATION UNIT ~74

↓

FEATURE AMOUNT CORRECTION UNIT ~76

↓

RESOLUTION REDUCTION PROCESSING UNIT ~77

↓

IMAGE GENERATION UNIT ~78

62 ALIGNMENT PROCESSING UNIT → 63 BRIGHTNESS CORRECTION PROCESSING UNIT →

## FIG. 15

LIGHT INTENSITY

FIRST LIGHT EMISSION PATTERN

V

400  450  500  550  600  650  700  WAVELENGTH (nm)

↕

LIGHT INTENSITY

SECOND LIGHT EMISSION PATTERN

G    R

400  450  500  550  600  650  700  WAVELENGTH (nm)

# FIG. 16

LIGHT INTENSITY

FIRST LIGHT EMISSION PATTERN

V

400  450  500  550  600  650  700  WAVELENGTH (nm)

↕

LIGHT INTENSITY

SECOND LIGHT EMISSION PATTERN

B  Bx  G  R

400  450  500  550  600  650  700  WAVELENGTH (nm)

# FIG. 17

20

| 102 | 101 |
|---|---|
| ROTATION FILTER | BROADBAND LIGHT SOURCE |

## FIG. 18

## FIG. 19

# INTERNATIONAL SEARCH REPORT

| International application No. |
|---|
| PCT/JP2017/025877 |

A. CLASSIFICATION OF SUBJECT MATTER
*A61B1/045*(2006.01)i, *A61B1/00*(2006.01)i, *G02B23/24*(2006.01)i, *H04N7/18* (2006.01)i

According to International Patent Classification (IPC) or to both national classification and IPC

B. FIELDS SEARCHED

Minimum documentation searched (classification system followed by classification symbols)
A61B1/00-1/32

Documentation searched other than minimum documentation to the extent that such documents are included in the fields searched
Jitsuyo Shinan Koho          1922-1996      Jitsuyo Shinan Toroku Koho    1996-2017
Kokai Jitsuyo Shinan Koho    1971-2017      Toroku Jitsuyo Shinan Koho    1994-2017

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | WO 2016/147435 A1  (Olympus Corp.), 22 September 2016 (22.09.2016), paragraph [0023] & US 2017/0027428 A1 paragraph [0063] & EP 3114985 A1 | 1-15 |
| A | JP 2016-77756 A  (Fujifilm Corp.), 16 May 2016 (16.05.2016), paragraph [0033] (Family: none) | 1-15 |
| A | JP 2014-166590 A  (Fujifilm Corp.), 11 September 2014 (11.09.2014), paragraph [0003] (Family: none) | 1-15 |

☐ Further documents are listed in the continuation of Box C.          ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 07 September 2017 (07.09.17) | 19 September 2017 (19.09.17) |

| Name and mailing address of the ISA/ Japan Patent Office 3-4-3,Kasumigaseki,Chiyoda-ku, Tokyo 100-8915,Japan | Authorized officer |
|---|---|
| | Telephone No. |

Form PCT/ISA/210 (second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016067775 A